(19)

**Europäisches Patentamt**
**European Patent Office**
**Office européen des brevets**

(11)  **EP 4 715 830 A1**

(12)  **EUROPEAN PATENT APPLICATION**

(43) Date of publication:
**25.03.2026 Bulletin 2026/13**

(21) Application number: **25192687.9**

(22) Date of filing: **30.07.2025**

(51) International Patent Classification (IPC):
**G16C 60/00** (2019.01)    *G16C 20/10* (2019.01)
*G16C 20/30* (2019.01)    *G16C 20/70* (2019.01)

(52) Cooperative Patent Classification (CPC):
**G16C 60/00;** G16C 20/10; G16C 20/30; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.09.2024 KR 20240129283**

(71) Applicants:
• **SK Innovation Co., Ltd.**
**Seoul 03188 (KR)**
• **SK Geo Centric Co., Ltd.**
**Seoul 03188 (KR)**

(72) Inventors:
• **KO, Suk Joon**
**34124 Daejeon (KR)**

• **KWON, Ky Sang**
**34124 Daejeon (KR)**
• **SHIN, Joo Hyun**
**34124 Daejeon (KR)**
• **KIM, Duk Hee**
**03161 Seoul (KR)**
• **KIM, Sun Ryong**
**03161 Seoul (KR)**
• **KWON, June Haan**
**03161 Seoul (KR)**
• **LEE, Joo Pyung**
**03161 Seoul (KR)**

(74) Representative: **Lorenz Seidler Gossel Part. mbB**
**Widenmayerstr. 23**
**80538 München (DE)**

(54)  **METHOD FOR GENERATING RECIPES OF POLYMER COMPOSITE MATERIAL AND DEVICE THEREOF**

(57)    A method for predicting recipes of a polymer composite material and a device thereof may be provided, wherein the method comprises: inputting a target property for at least two property items related to a target polymer composite material and a constraint condition for use of a material related to synthesis of the target polymer composite material; generating a predicted recipe for synthesis of the target polymer composite material using a recipe prediction model under the input target property and the constraint condition; and outputting the generated predicted recipe; wherein the predicted recipe comprises at least two materials and a mixing ratio for the at least two materials.

EP 4 715 830 A1

**Description**

<u>CROSS-REFERENCE TO RELATED APPLICATION</u>

This application claims priority to Korean Patent Application No. 10-2024-0129283 filed September 24, 2024, the disclosure of which is hereby incorporated by reference in its entirety.

<u>BACKGROUND</u>

<u>Technical Field</u>

The present disclosure relates to a method for generating recipes of a polymer composite material and a device thereof.

<u>Technical Considerations</u>

A polymer composite material is used in various industrial fields, and in order to predict properties according to recipes, or predict materials of a polymer composite material having target properties during the development and synthesis thereof, experiments, simulations, and data-based approaches are comprehensively utilized.

The current techniques use methods that still require repeated trial and error methods in order to predict materials for the polymer composite material with target properties, for example, a method of collecting property data by trying the prediction while changing various composition ratios and process conditions in a laboratory, inferring the properties of the material based on the collected property data, and improving the mixing of materials to secure a composition of the materials is used.

Thereby, attempts are being made to predict materials for the polymer composite material with target properties using a model trained based on the artificial intelligence currently developed, but it takes a lot of costs and time to perform training of the commercially available artificial intelligence model, and there are limitations due to a reason that it is difficult to consider numerous variables and interactions between recipes and properties of the material synthesized based on the recipes.

<u>SUMMARY</u>

It is an object of the present disclosure to provide a method for generating recipes of a polymer composite material and a device thereof.

Problems to be solved through various embodiments are not limited to the above-described problem, and other problems not described above will be clearly understood by those skilled in the art from the following description.

To achieve the above object, according to some non-limiting embodiments or aspects of the present disclosure, there is provided a method for generating recipes of a polymer composite material, which comprises: inputting a target property for at least two property items related to a target polymer composite material and a constraint condition for use of a material related to synthesis of the target polymer composite material; generating a predicted recipe for synthesis of the target polymer composite material using a recipe prediction model under the input target property and the constraint condition; and outputting the generated predicted recipe; wherein the predicted recipe comprises at least two materials and a mixing ratio for the at least two materials.

In some non-limiting embodiments or aspects, the recipe prediction model is configured to generate the predicted recipe based on a property prediction model which is pre-trained using artificial intelligence and an optimization algorithm applied to the property prediction model, and the property prediction model is an artificial intelligence model trained to receive as an input, an input recipe comprising a plurality of materials comprising at least one polymer and a mixing ratio for the plurality of materials, and generate predicted properties of the polymer composite material according to an input of the input recipe.

In some non-limiting embodiments or aspects, generating the predicted recipe comprises: generating a search space and a grid based on the search space for deriving candidate recipes based on the target property, the constraint condition, and the property prediction model; dividing the search space into a plurality of search areas based on materials having linearly approximable properties and the property prediction model; reducing the plurality of search areas based on a preset weight; and determining, as the predicted recipe, candidate recipes calculated by applying the optimization algorithm in parallel to each of the reduced plurality of search areas.

In some non-limiting embodiments or aspects, reducing the plurality of search areas comprises: determining search areas in which an optimal solution exists, using an objective function generated based on a result to which the weight is applied for a grid point of each of the plurality of search areas; and obtaining the reduced plurality of search areas configured with the search areas in which the optimal solution exists.

In some non-limiting embodiments or aspects, reducing the plurality of search areas comprises, changing at least some values of the constraint condition when a number of the search areas in which the optimal solution exists is less than a preset number.

In some non-limiting embodiments or aspects, the target property comprises at least one of a property value range for at least one property item or information on candidate materials.

In some non-limiting embodiments or aspects, the constraint condition comprises information on at least one of: at least one required material, a minimum usage ratio for each material, or a number of maximum usable materials.

In some non-limiting embodiments or aspects, determining the predicted recipe further comprises: obtaining at least one updated objective function for deriving the candidate recipes during applying the optimization algorithm in parallel to each of the reduced plurality of search areas; calculating an error of the at least one updated objective function; and excluding, from the reduced plurality of search areas, a search area corresponding to an updated objective function having an error equal to or greater than a preset value.

In some non-limiting embodiments or aspects, there is provided a device for generating recipes of a polymer composite material, the device comprising: a property information processing unit configured to input a target property for at least two property items related to a target polymer composite material and a constraint condition for use of a material related to synthesis of the target polymer composite material; a recipe prediction processing unit configured to generate a predicted recipe for synthesis of the target polymer composite material using a recipe prediction model under the input target property and the constraint condition; and a result output unit configured to output the generated predicted recipe; wherein the predicted recipe comprises at least two materials and a mixing ratio for the at least two materials.

In some non-limiting embodiments or aspects, the recipe prediction model is configured to generate the predicted recipe based on a property prediction model which is pre-trained using artificial intelligence and an optimization algorithm applied to the property prediction model, and the property prediction model is an artificial intelligence model trained to receive as an input, an input recipe comprising a plurality of materials comprising at least one polymer and a mixing ratio for the plurality of materials, and generate predicted properties of the polymer composite material according to an input of the input recipe.

In some non-limiting embodiments or aspects, the recipe prediction processing unit is configured to: generate a search space and a grid based on the search space for deriving candidate recipes based on the target property, the constraint condition, and the property prediction model; divide the search space into a plurality of search areas based on materials having linearly approximable properties and the property prediction model; reduce the plurality of search areas based on a preset weight; and determine, as the predicted recipe, candidate recipes calculated by applying the optimization algorithm in parallel to each of the reduced plurality of search areas.

In some non-limiting embodiments or aspects, the recipe prediction processing unit is configured to: determine search areas in which an optimal solution exists, using an objective function generated based on a result to which the weight is applied for a grid point of each of the plurality of search areas; and obtain the reduced plurality of search areas configured with the search areas in which the optimal solution exists.

In some non-limiting embodiments or aspects, the recipe prediction processing unit is configured to change at least some values of the constraint condition when a number of the search areas in which the optimal solution exists is less than a preset number, while performing an operation of reducing the plurality of search areas.

In some non-limiting embodiments or aspects, the target property comprises at least one of a property value range for at least one property item or information on candidate materials.

In some non-limiting embodiments or aspects, the constraint condition comprises information on at least one of: at least one required material, a minimum usage ratio for each material, or a number of maximum usable materials.

In some non-limiting embodiments or aspects, the recipe prediction processing unit is configured to: obtain at least one updated objective function for deriving the candidate recipes during applying the optimization algorithm in parallel to each of the reduced plurality of search areas; calculate an error of the at least one updated objective function; and exclude, from the reduced plurality of search areas, a search area corresponding to an updated objective function having an error equal to or greater than a preset value..

In some non-limiting embodiments or aspects, the method and the device for generating recipes of a polymer composite material may provide an environment capable of quickly deriving recipes which is satisfied with the target property and the constraint condition by deriving the predicted recipe based on a Bayesian optimization algorithm applying in parallel to a plurality of targets (the search areas).

In some non-limiting embodiments or aspects, the method and the device for generating recipes of a polymer composite material may improve a matching rate of a recipe for the target property and the constraint condition by controlling flexibly ranges of the search areas based on an error in a process of Bayesian optimization.

In some non-limiting embodiments or aspects, by determining search areas in which an optimal solution exists, using an objective function generated based on a result to which the weight is applied for a grid point of each of the plurality of search areas, the method and the device for generating recipes of a polymer composite material may reduce a situation that a recipe which is not satisfied with a real target property is derived due to an overfitting of the property prediction model which is pre-trained using artificial intelligence, or a prediction error.

BRIEF DESCRIPTION OF THE DRAWINGS

The above and other objects, features and other advantages of the present disclosure will be more clearly understood from the following detailed description taken in conjunction with the accompanying drawings, in which:

FIG. 1 is a block diagram schematically illustrating the configuration of a device according to a non-limiting embodiment according to the principles of the present disclosure;
FIG. 2 is a flowchart illustrating the flow of an operation of predicting recipes for a polymer composite material with the target property in the device according to a non-limiting embodiment according to the principles of the present disclosure;
FIG. 3 is a flowchart illustrating the detailed flow of the operation of predicting recipes for a polymer composite material with the target property using the recipe prediction model in the device according to a non-limiting embodiment according to the principles of the present disclosure;
FIG. 4 is a flowchart illustrating an operation of reducing search areas for applying the optimization algorithm of the recipe prediction model in the device according to a non-limiting embodiment;
FIG. 5 is a flowchart illustrating the detailed flow of the operation of applying the optimization algorithm of the recipe prediction model in the device according to a non-limiting embodiment;
FIG. 6 is a diagram illustrating a grid generated to display a possibility of feasible solutions of the search space, based on hyper-parameters of the constraint conditions input to the recipe prediction model in the device according to a non-limiting embodiment;
FIG. 7 is a view schematically illustrating an operation of excluding the search area in a process that the Bayesian optimization algorithm is applied in parallel in the device according to a non-limiting embodiment.

DETAILED DESCRIPTION

Hereinafter, embodiments or aspects will be described in detail with reference to the accompanying drawings. However, since various changes may be made in the embodiments or aspects, the scope of the patent disclosure is not limited or restricted by these embodiments or aspects. It should be understood that all modifications, equivalents, and alternatives for the embodiments or aspects are comprised in the scope of the present disclosure.

It will be understood that when a component is described to as being "connected," "combined" or "coupled" to another component, the component may be directly connected or coupled to the another component, but it may be "connected," "combined" or "coupled" to the another component by an intervening another component that may be present.

Further, in describing the components of the embodiment or aspects, the meaning of "or" may mean each of the components, may mean two or more of the components, or may mean all of the components. For example, it should be understood that the expressions "a, b or c" represent any one of "a," "b," "c," "a and b," "a and c," "b and c," and "a, b and c."

Components comprised in one embodiment or aspects and components comprising common functions will be described using the same names in other embodiments or aspects. The description given in one embodiment or aspects may be applied to other embodiments or aspects, and therefore will not be described in detail within the overlapping range, unless there is a description opposite thereto.

The device and/or 'data' processed by the device may be expressed in terms of "information". Here, the information may be used as a concept comprising the data.

The present disclosure describes a method for generating recipes of a polymer composite material and a device thereof. To describe in more detail, the present disclosure relates to a method for generating recipes (e.g., predicted recipes) of a polymer composite material corresponding to a target property and a device thereof, and it may be described a method and a device for generating recipes of a target polymer composite material which satisfy the target property and/or the constraint condition input from a user.

According to various non-limiting embodiments or aspects, the operation of generating recipes of a polymer composite material may be performed based on at least one deep learning algorithm. To describe in more detail, the operation of generating recipes of a polymer composite material may be performed through a recipe prediction model, and the recipe prediction model may be configured to output a predicted recipe corresponding to the target property based on an artificial intelligence model (a deep learning model) trained based on a deep learning algorithm.

In some non-limiting embodiments or aspects, the deep learning model may comprise a property prediction model trained to predict properties of a target polymer composite material generated for a recipe (e.g., an input recipe) comprising a plurality of materials including polymers, properties of the materials, and a mixing ratio of the materials.

In some non-limiting embodiments or aspects, the recipe prediction model for generating recipes of the target polymer composite material, which satisfy the target property, may comprise an optimization algorithm, and may be configured to output the predicted recipe from the target property by applying the optimization algorithm to the property prediction model.

For example, the recipe prediction model may be configured to generate the predicted recipe based on a property prediction model which is pre-trained using artificial intelligence and an optimization algorithm applied to the property prediction model.

Here, the machine learning algorithm may comprise at least some of optimization algorithms based on machine learning, such as a Bayesian optimization algorithm, a grid search algorithm, and a gradient descent algorithm.

Hereinafter, various embodiments or aspects for generating recipes of the target polymer composite material corresponding to the target property based on the recipe prediction model comprising the Bayesian optimization algorithm may be described.

In some non-limiting embodiments or aspects, the polymer composite material may be a material prepared using at least one polymer, as well as polymer blends, polymer copolymer, polymer nanocomposites, polymer interpenetrating network (IPN), and/or polymer metal composites.

In addition, the polymer which is the material of the polymer composite material may comprise at least one polymer selected from polypropylene (PP), polyethylene (PE), and/or polyethylene terephthalate (PET).

In this case, the polymer may comprise polypropylene, polyethylene, and/or polyethylene terephthalate as polymer categories, and may comprise at least one polymer material for each of these categories.

Hereinafter, various embodiments or aspects of the present disclosure will be described with reference to the accompanying drawings. However, the drawings attached to the present specification serve to further understand the technical idea together with the detailed description, such that the present disclosure should not be construed as being limited only to the illustrations of the drawings.

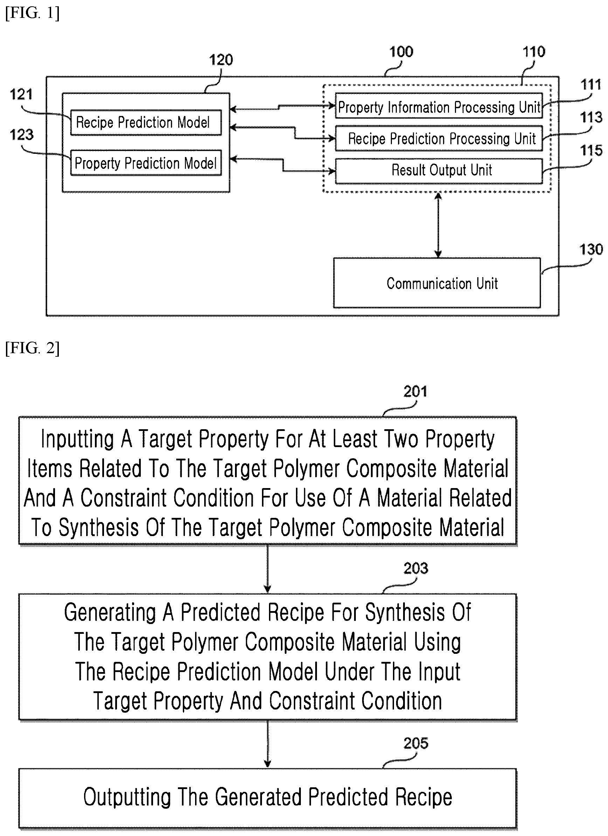

FIG. 1 is a block diagram illustrating the configuration of a device according to some non-limiting embodiments or aspects. To describe in more detail, the device may be illustrated as a block diagram by dividing the detailed configuration according to functions thereof as shown in FIG. 1.

First, referring to FIG. 1, a device 100 may comprise at least some of a processing unit 110, a storage unit 120, and a communication unit 130.

The processing unit 110 may comprise at least one processor, and may process data through at least one program (application, tool, plug-in, software, etc.) (e.g., a recipe prediction program).

The processing unit 110 may be configured to predict a recipe (a predicted recipe) capable of synthesis (or compounding) of a polymer composite material corresponding to a property using an input property (a target property).

To this end, the processing unit 110 may comprise a property information processing unit 111 configured to obtain at least some of target properties and constraint conditions of a target polymer composite material, a recipe prediction processing unit 113 configured to obtain a predicted recipe based on at least one target property for a target polymer composite material to be synthesized, constraint conditions and the recipe prediction model 121, and a result output unit 115 configured to output the predicted recipe.

Here, although the property information processing unit 111, the recipe prediction processing unit 113, and the result output unit 115 are illustrated and described as being included in the processing unit 110, the present disclosure is not limited thereto, and at least some of the property information processing unit 111, the recipe prediction processing unit 113, and the result output unit 115 may be included in the device 100 as separate elements from the processing unit 110.

Here, at least two of the property information processing unit 111, the recipe prediction processing unit 113, and the result output unit 115 may be configured as one module, or each of them may be configured as a separate module. In this case, each module may comprise at least one processor.

Hereinafter, functions of various embodiments described as being performed by the device 100 may be described as operations processed through the property information processing unit 111, the recipe prediction processing unit 113, or the result output unit 115, operations processed through a module configured to include at least some of the property information processing unit 111, the recipe prediction processing unit 113, and the result output unit 115, or operations processed through the processing unit 110.

In addition, functions of various embodiments or aspects described as being performed by the device 100 may be performed in conjunction with components of the device 100 or other devices connected with the device 100.

The storage unit 120 may store various data processed by at least one component of the device 100 (e.g., the processing unit 110 or the communication unit 130, etc.). The data may include, for example, a program for processing control commands, data processed through the program, or input data and output data related thereto.

To describe in more detail, the storage unit 120 may comprise an artificial neural network algorithm, a blockchain algorithm, a deep learning algorithm, a regression analysis algorithm, and related mechanisms, operators, language models, and big data, which are for processing control commands.

The storage unit 120 may include a recipe prediction model 121 configured to predict materials including at least one polymer and mixing ratios of the materials for generation (e.g., compounding) of the polymer composite material that satisfies the input target property.

According to an embodiment, the recipe prediction model 121 may be configured to predict a recipe when the target property of the polymer composite material is input, and may be configured to use (or comprise) an optimization algorithm.

To describe in more detail, the recipe prediction model 121 may comprise an optimization algorithm configured to predict a recipe that satisfies the input target property and constraint condition.

Here, the recipe of the polymer composite material (e.g., a predicted recipe predicted through the recipe prediction model 121) may be configured to comprise at least two materials including at least one polymer for synthesis of the polymer composite material and a mixing ratio for each of the at least two materials.

In this case, the predicted recipe predicted through the recipe prediction model 121 may be configured to satisfy the input constraint condition.

Here, the recipe prediction model 121 operates using the property prediction model 123, and for this, the storage unit 120 may store the property prediction model 123 generated and trained to predict properties of the polymer composite material, the generation of which is predicted according to the input recipe.

Referring to FIG. 1, although the recipe prediction model 121 and the property prediction model 123 are illustrated as being stored in the storage unit 120 as independent models, respectively, the present disclosure is not limited thereto, and at least a portion of the property prediction model 123 may be included in the recipe prediction model 121.

The property prediction model 123 may be an artificial intelligence model trained to predict properties of the polymer composite material according to a recipe based on properties of materials included in the recipe and mixing ratios of the materials, when the recipe is input.

Here, the recipe input to the property prediction model 123 may include a plurality of materials including at least one polymer and a mixing ratio for the plurality of materials, as described above.

Here, the artificial intelligence model may be a model trained based on artificial intelligence such as deep learning, machine learning, and may be configured based on at least some of various deep learning algorithms that process structured data, such as TabNet, XGBoost, LightGBM, CatBoost, and Deep Neural Network (DNN).

For this, although not illustrated in FIG. 1, the storage unit 120 may be configured to include a training dataset (not illustrated) for training of the property prediction model 123.

Here, the training data may include a training recipe for at least one training polymer composite material and training property information. Here, the training recipe may be configured to include information on materials including at least one polymer and mixing ratios of the materials so as to satisfy the properties of each of the at least one training polymer composite material, as described above.

The communication unit 130 may support establishment of a wired communication channel inside the device 100 and/or between the device 100 and at least one other device (e.g., a user device or a server), establishment of a wireless communication channel, and performing communication through the established communication channel.

Although not illustrated in the drawings, the device 100 may be configured to further include at least one input/output unit.

According to an embodiment, the input/output unit may include or be connected to at least some of an input unit (not illustrated) that inputs data, such as a keyboard, a mouse, and a touch pad, and an output unit (not illustrated) that outputs data, such as a display unit (e.g., a display) and a speaker.

According to various embodiments of the present disclosure, the device 100 or the user device may include at least some of functions of a range of all information communication devices including a mobile communication terminal, a multimedia terminal, a wired terminal, a fixed terminal, and an internet protocol (IP) terminal.

The device 100 is a device for processing control commands, and may be configured to include or be connected through communication to at least some functions of a workstation or a large-capacity database.

Hereinafter, as an operation of the device 100 based on FIG. 1, an operation of predicting, by the device 100, a recipe for synthesizing a target polymer composite material that satisfies a preset target property may be described with reference to FIGS. 2 to 7.

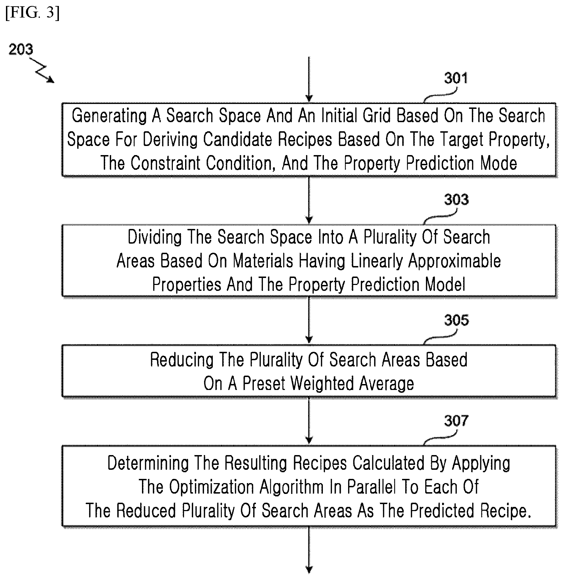

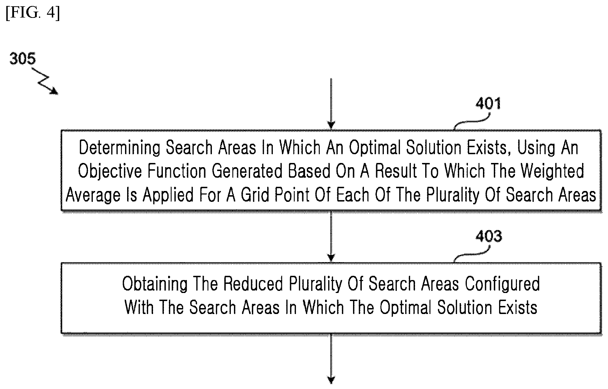

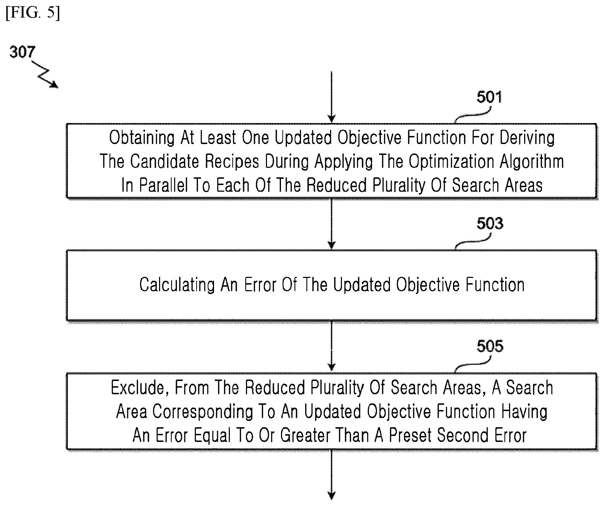

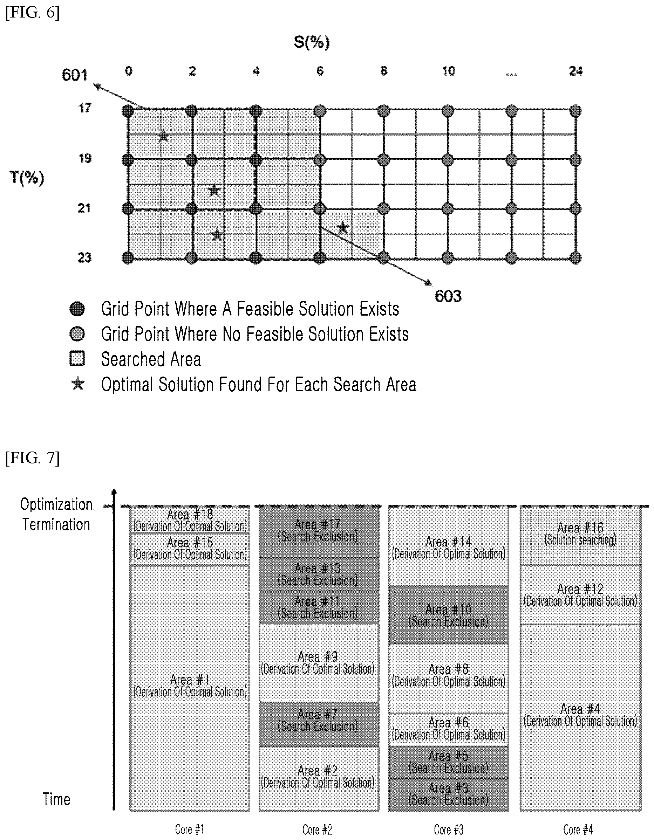

To this end, FIG. 2 is a flowchart illustrating the flow of an operation of predicting recipes for a polymer composite material with the target property in the device according to a non-limiting embodiment. FIG. 3 is a flowchart illustrating the detailed flow of the operation of predicting recipes for a polymer composite material with the target property using the recipe prediction model in the device according to a non-limiting embodiment. FIG. 4 is a flowchart illustrating the detailed flow of an operation of reducing search areas for applying the optimization algorithm of the recipe prediction model in the device according to a non-limiting embodiment. FIG. 5 is a flowchart illustrating the detailed flow of the operation of applying the optimization algorithm of the recipe prediction model in the device according to a non-limiting embodiment. FIG. 6 is a diagram illustrating a grid generated to display a possibility of feasible solutions of the search space, based on hyper-parameters of the constraint conditions input to the recipe prediction model in the device according to a non-limiting embodiment.

First, referring to FIG. 2, in step 201, the property information processing unit 110 may input a target property for at least two property items related to the target polymer composite material and a constraint condition for use of a material related to synthesis of the target polymer composite material.

For example, the property information processing unit 110 may obtain the target property and the constraint condition input through an input unit (or an input/output unit) of the device 100, or may receive the target property and the constraint condition through the communication unit 130.

Here, the target property may comprise at least one of at least one property item, a value for the at least one property item, and information on candidate materials.

For example, the property items may comprise at least one item of melt index (MI), tensile strength (TS), tensile break, tearing strength, yield strength, flexural modulus (FM), impact strength, elongation at break, heat distortion temperature, air permeability, and shrinkage.

To describe in more detail, the impact strength may comprise properties of dart impact strength and/or IZOD impact strength (IZOD). Also, at least some of tearing strength, yield strength, tensile break, and elongation at break may comprise properties in a machine direction and/or properties in a transverse direction.

In describing embodiments of the present disclosure, when expressing 'properties (or at least one property) of a specific material', for example, it may be expressed to include properties (or property items) and property values of the specific material, such as property items (e.g., tensile strength) and property values (tensile strength values possessed by a specific polypropylene) of the specific material (e.g., specific polypropylene).

According to an embodiment, the value for each of the obtained at least one property item may be obtained as a maximum value or a minimum value. For example, the recipe prediction processing unit 113 may obtain a target property comprising a minimum value of 25 MPa in a tensile strength item, a minimum value of 0.2 g/10 min in a melt index item, a minimum value of 80 degrees Celsius (°C) in a heat distortion temperature item, and a minimum value of 1.5% in a shrinkage item, for at least one property item.

However, without being limited thereto, the value for at least one item among the obtained at least one property item may also be obtained as a range.

Information on candidate materials may include at least one property item and a property value for the property item that at least one candidate material has. Here, the information on the candidate materials may be included in the target property obtained by the property information processing unit 110, as described above. However, without being limited thereto, it may be in a state stored in the storage unit 120.

Here, the candidate materials may be configured to include materials that can be supplied for synthesis of the polymer composite material.

In addition, the constraint condition may include information on at least one of: at least one required material, a minimum usage ratio for each material, a number of maximum usable materials, and at least some of controllable values for ratios of materials included in the predicted recipe.

To describe in more detail, the constraint condition may include information on at least one of: at least one required material to be included in the predicted recipe of the target polymer synthetic resin, a minimum usage ratio of a required material or a material included in the recipe, a maximum usage ratio of a required material or a material included in the recipe, a maximum usable number of materials, controllable minimum units (or minimum values) for at least some of the ratios of the materials included in the predicted recipe, and controllable ranges for at least some of the ratios of the materials included in the predicted recipe.

In step 203, the recipe prediction processing unit 113 may generate a predicted recipe for synthesis of the target polymer composite material using the recipe prediction model 121 under the input target property and constraint condition.

Here, the predicted recipe may comprise at least two materials and a mixing ratio for the at least two materials. Here, the material comprises at least one polymer, and may be configured to further include at least some of talc, polyolefin elastomer, and polyolefin ether.

For example, the predicted recipe may be configured to include, as materials capable of generating the target polymer composite material that satisfies at least some properties and constraint conditions obtained as described above, at least one polymer, a reinforcing agent (e.g., talc), and an additive (e.g., polyolefin ether, etc.), and a mixing ratio thereof.

Here, the mixing ratio may be represented as a relative weight ratio of each material (e.g., weight ratio sum, WRS), and the content for each material may be represented as %.

In addition, the predicted recipe may also include at least some of property (material property) items for each of the included materials and property values (material property values) for the properties (material properties).

To this end, the storage unit 120 may store, as a database, various materials that can be used for synthesis of the polymer composite material, and information on the materials (e.g., property information).

In addition, at least some of the two or more materials included in the recipe output from the recipe prediction model 121 may include identification information (e.g., code, ID, etc.) of the corresponding material and/or a property value for at least one property item.

Here, the recipe prediction model 121 may generate a predicted recipe by including identification information and/or property values for at least one property item for at least some of the at least two materials included in the predicted recipe, in order to specify any one material among various materials having the same or similar properties.

For example, the polymer may include not only normal (or virgin) polymers, but also at least one of various recycled polymers such as recycled polypropylene (rPP), recycled polyethylene (rPE), and recycled polyethylene terephthalate (rPET).

In this case, even if it is the same polymer, especially in the case of recycled polymers, the properties may not be uniform depending on the time of production.

In order to distinguish the same material in the case where there is a difference in properties as described above, the recipe may include identification information for materials including a polymer and/or a property value for at least one property item that can represent characteristics of the material.

The constraint condition may include a specific polymer (e.g., virgin polymer, recycled polymer, or polymer identification information, etc.) and/or a mixing ratio (value or range) of the specific polymer to be included in the predicted recipe among various polymers as described above.

According to the above description, the recipe prediction processing unit 113 may derive a predicted recipe from the input target property and constraint condition based on the optimization algorithm of the recipe prediction model 121.

Here, the optimization algorithm includes at least a part of a Bayesian optimization algorithm, generates an objective function based on the property prediction model 123, and may output the predicted recipe as a decision variable by applying the input target property and constraint condition to the objective function as independent variables.

Here, the objective function may be included in the recipe prediction model 121, which is pre-generated for recipe prediction according to various embodiments of the present disclosure. However, without being limited thereto, the objective function may be generated and changed through a process in which the recipe prediction processing unit 113 derives a predicted recipe.

Here, even if the target property and/or constraint condition include information on the IZOD impact strength, the objective function may be modeled excluding the variable for the IZOD impact strength. Based on this, it is possible to prevent modeling uncertainty of the objective function due to the nonlinearity of the property prediction model 123.

In addition, the recipe prediction model 121 may be configured to include at least one relational equation that calculates a difference in properties between a virgin polymer and a recycled polymer for the same at least one polymer.

Hereinafter, an operation of generating the predicted recipe based on the recipe prediction model 121 and the input target property and constraint condition may be described in more detail with reference to FIGS. 3 to 5.

First, referring to FIG. 3, in step 301, the recipe prediction processing unit 113 may generate a search space and an initial grid based on the search space for deriving candidate recipes based on the target property, the constraint condition, and the property prediction model.

According to an embodiment, the recipe prediction processing unit 113 may form a search space for optimizing an objective function based on the target property and the constraint condition, and may generate an initial grid for the search space.

To describe in more detail, when a material having a linearly approximable property (hereinafter, a linearly approximable material) is included in the constraint condition, the recipe prediction processing unit 113 may generate an initial grid based on the linearly approximable material.

In step 303, the recipe prediction processing unit 113 may divide the search space into a plurality of search areas based on materials having linearly approximable properties and the property prediction model 123.

To describe in more detail, the constraint condition may include a plurality of linearly approximable materials, and as illustrated in FIG. 6, the recipe prediction processing unit 113 may set a search area for an intersection point or grid point based on ratios of at least two linearly approximable materials (S and T).

Here, the recipe prediction processing unit 113 may set a surrounding area of the grid point as the search area. To describe in more detail, the search area may be set to include at least one unit area centering on the grid point.

In step 305, the recipe prediction processing unit 113 may reduce the plurality of search areas based on a preset weight (e.g., a weighted average) and set the reduced plurality of search areas.

To describe in more detail, the recipe prediction processing unit 113 may set the reduced plurality of search areas including search areas of grid points in which an optimal solution exists according to whether an optimal solution exists or can be derived for the grid points or an optimal solution search result.

Here, referring to FIG. 6, the recipe prediction processing unit 113 may set the grid point as a grid point where a feasible solution exists when an optimal solution can be derived for the grid point. On the other hand, the recipe prediction processing unit 113 may set the grid point as a grid point where no feasible solution exists when an optimal solution cannot be derived for the grid point.

The recipe prediction processing unit 113 may set the search areas of the grid points where feasible solutions exist as the reduced plurality of search areas.

In this regard, referring to FIG. 4, to describe in more detail, first, in step 401, the recipe prediction processing unit 113 may apply a weight to the grid point and then perform an optimal solution search for the grid point.

Here, the weight applied to the grid point may be for solving a linear approximation problem of adjacent grid points, and may be applied to at least one property item constituting the grid point.

For example, a linear optimization function including an approximate property prediction formula by a weight (e.g., a weighted average) applied to a grid point may be configured as in Equation (1).

$$\min max \left( MI_k^T \cdot x - MI_{UB}, 0 \right) - \left( FM_k^T \cdot x - FM_{LB} \right) + \left( TS_k^T \cdot x - TS_{LB} \right) \quad (1)$$

Here,

$$MI_k^T \cdot x \geq MI_{LB}, \quad FM_k^T \cdot x \geq FM_{LB}, \quad TS_k^T \cdot x \geq TS_{LB}, \quad IZOD_k^T \cdot x \geq IZOD_{LB}, \quad \sum x = 1$$

are satisfied, and x is a mixing ratio, MI is a melt index, FM is a flexural modulus, TS is tensile strength, IZOD is impact strength, $MI_k^T \cdot x$ is an approximate property prediction formula for a melt index, $FM_k^T \cdot x$ is an approximate property prediction formula for a flexural modulus, $TS_k^T \cdot x$ is an approximate property prediction formula for tensile strength, $IZOD_k^T \cdot x$ is an approximate property prediction formula for impact strength, UB (upper bound) may be described as a maximum value (or an upper limit value), and LB (low bound) may be described as a minimum value (or a lower limit value).

The recipe prediction processing unit 113 may perform an optimal solution search for the grid points in the linear optimization function to which the weight (weighted average) according to the difference in the mixing ratio of the property items is applied, and in step 403, may obtain (or set) the search areas of the grid points where the optimal solution exists as the reduced plurality of search areas.

Here, the optimal solution search may be described as deriving an optimal solution that minimizes or maximizes the objective function for a grid point to which a weight is applied in relation to linear programming.

The recipe prediction processing unit 113 may set the optimal solution of each of the reduced plurality of search areas as an initial solution of the corresponding search area.

For example, referring to FIG. 6, the recipe prediction processing unit 113 may derive an optimal solution for a first search area 601 of a grid point where the ratio of the first linearly approximable material (S) is 2% and the ratio of the second linearly approximable material (T) is 19%, and set the derived optimal solution as an initial solution of the first search area 601.

As another example, the recipe prediction processing unit 113 may derive an optimal solution for a second search area 603 of a grid point where the ratio of the first linearly approximable material (S) is 4% and the ratio of the second linearly approximable material (T) is 21%, and set the derived optimal solution as an initial solution of the second search area 603.

Returning to step 305 of FIG. 3 again, the recipe prediction processing unit 113 may perform step 307 when the reduced plurality of search areas satisfy a preset number. On the other hand, the recipe prediction processing unit 113 may repeatedly perform an optimal solution search by applying a changed weight to grid points where an optimal solution does not exist, when the reduced plurality of search areas do not satisfy the preset number.

To describe in more detail, the recipe prediction processing unit 113 may change (e.g., loosen) a condition for at least one property in Equation (1) for applying the weight, and based on this, may repeatedly perform an operation of searching for an optimal solution for a grid point (a grid point where an optimal solution does not exist) to which the changed weight is applied.

Based on this, the recipe prediction processing unit 113 may additionally obtain search areas for grid points in which an optimal solution exists until the number of the reduced plurality of search areas reaches (or is equal to or greater than) the preset number.

Here, the preset number for the reduced plurality of search areas may be set according to the number (N) of predicted recipes set to be generated using the recipe prediction model 121.

For example, when the number of predicted recipes set to be generated is 10, the recipe prediction processing unit 113 may perform application of the changed weight and optimal solution search as described above until the number of reduced plurality of search areas set by optimal solution search is 10 or more.

In step 307, the recipe prediction processing unit 113 may determine the resulting recipes calculated by applying the optimization algorithm in parallel to each of the reduced plurality of search areas as the predicted recipe.

To describe in more detail, the recipe prediction processing unit 113 may apply the optimization algorithm to each of the reduced plurality of search areas, and may derive at least one candidate recipe based on an initial value set for each of the reduced plurality of search areas.

To describe the process of deriving a candidate recipe in more detail, first, the recipe prediction processing unit 113 may initialize the objective function based on an initial solution (e.g., $x_{1:n-1}$) of each of the reduced plurality of search areas, and may obtain the initialized objective function (e.g., $f(x_{1:n-1})$).

The recipe prediction processing unit 113 may derive a candidate recipe that satisfies the target property and the constraint condition based on the initialized objective function (e.g., $f(x_{1:n-1})$). Here, the recipe prediction processing unit 113 may derive at least one candidate recipe by repeatedly performing an operation of deriving an optimal solution based on the initialized objective function and the optimization algorithm.

At this time, the recipe prediction processing unit 113 may update at least some parameters of the objective function in repeatedly performing the operation of deriving the optimal solution (candidate recipe) of the objective function based on the optimization algorithm, and may derive the optimal solution (candidate recipe) based on the updated objective function.

Describing in more detail on the assumption of the initial solution $x_{n-1}$, the recipe prediction processing unit 113 may model a posterior probability distribution (or a surrogate function) for the initialized objective function $f(x_{n-1})$.

For example, the recipe prediction processing unit 113 may model a relationship between the input variable $x_{n-1}$ and the output variable $f(x_{n-1})$ based on a Gaussian Process Regression (GPR), and may model a distribution function by providing an average and a variance of the objective function for the input value.

Here, the recipe prediction processing unit 113 may model the distribution function (or the surrogate function) using at least some of the target property and the constraint condition.

The recipe prediction processing unit 113 may search for $\widehat{x_n}$ that is the maximum solution of the distribution function (or surrogate function), and derive $\widehat{x_n'}$ to satisfy the constraint condition (and/or target property) which is a hyperparameter.

Thereafter, the recipe prediction processing unit 113 may derive normalized $x_n$ to satisfy $\sum \widehat{x_n'} = 1$, and update the objective function by obtaining the objective function $f(x_n)$ based on $x_n$.

Here, the recipe prediction processing unit 113 may perform an operation of deriving $x_n$ based on at least one acquisition function, for example, an expected improvement (EI) function.

The recipe prediction processing unit 113 may repeatedly perform the operation of improving the objective function and deriving a candidate recipe as described above, and may apply it in parallel to each of the reduced plurality of search areas.

The recipe prediction processing unit 113 may repeatedly perform the operation of optimizing the objective function and deriving a candidate recipe as described above until an error of $f(x_n)$ is equal to or less than a preset first error (e.g., $\varepsilon 1$) (e.g., $f(x_n) < \varepsilon 1$ ).

At this time, the recipe prediction processing unit 113 may perform operations such as reducing a search area by changing at least some of the objective function and/or the constraint condition, or optimizing the objective function and deriving a candidate recipe.

For example, the recipe processing unit 113 may extend the range of melt index (MI) to +5% of the value (or range) set in the target property (or constraint condition). Here, +5% may be changed according to the setting.

In addition, as described above, the recipe processing unit 113 may extend the range of melt index, tensile strength (or tear strength) to a preset limit value of the value (or range) set in the target property (or constraint condition).

In addition, the recipe prediction processing unit 113 may optimize the reduced plurality of search areas in repeatedly performing the operation of optimizing the objective function and deriving a candidate recipe.

In this regard, it may be described with reference to FIG. 5.

In step 501, the recipe prediction processing unit 113 may obtain at least one updated objective function for deriving the candidate recipes during applying the optimization algorithm in parallel to each of the reduced plurality of search areas, as described above.

Here, the optimization algorithm may comprise an optimization algorithm (a Bayesian optimization algorithm) based on a Bayesian technique, as described above.

Thereafter, in step 503, the recipe prediction processing unit 113 may calculate an error of the updated objective function.

Thereafter, in step 505, the recipe prediction processing unit 113 may exclude, from the reduced plurality of search areas, a search area corresponding to an updated objective function having an error equal to or greater than a preset second error (e.g., $\varepsilon 2$).

Here, the unit of error may be expressed as a percentage (%). Also, the first error ($\varepsilon 1$) may be set to a value smaller than the second error ($\varepsilon 2$).

According to the above description, the operation of excluding a search area based on an error in the reduced plurality of search areas was described as being performed on the updated objective function, but the recipe prediction processing unit 113 may also perform it based on the initial objective function of each of the reduced plurality of search areas.

In performing the operation of FIG. 5, it may be described with reference to FIG. 7. Thus, FIG. 7 is a diagram schematically illustrating an operation of excluding a search area in a process of applying the Bayesian optimization algorithm in parallel, in apparatus according to an embodiment.

Referring to FIG. 7, the recipe prediction processing unit 113 may stop the optimization operation for areas where the result of the objective function is not good, and exclude the search area from the search range, thereby efficiently managing resources when applying the optimization algorithm in parallel.

Returning to step 307 of FIG. 3 again, the recipe prediction processing unit 113 may determine the obtained candidate recipes as the predicted recipe and terminate the execution of step 307 (and step 203) when the number of candidate recipes satisfies a preset number (N).

At this time, when the number of candidate recipes obtained is less than the preset number (N), that is, when the number of candidate recipes having an error within the first error (ε1) is less than the preset number (N), the recipe prediction processing unit 113 may repeatedly perform an operation of deriving candidate recipes until the number of candidate recipes satisfies the preset number(N), by adding search areas.

For example, the recipe prediction processing unit 113 may perform an operation of deriving candidate recipes by sequentially restoring search areas in the order of lower error among the search areas excluded based on the error, with priority, to the reduced plurality of search areas.

In addition, even when the number of candidate recipes obtained is less than the preset number (N) after a preset first time has elapsed, the recipe prediction processing unit 113 may repeatedly perform an operation of deriving candidate recipes until the number of candidate recipes satisfies the preset number (N), by adding search areas as described above.

In addition, when the number of candidate recipes obtained is less than the preset number (N) after a preset second time has elapsed, the recipe prediction processing unit 113 may determine the obtained candidate recipes as the predicted recipe and terminate the execution of step 307 (and step 203).

Here, the first time may be set to be shorter than the second time.

Returning to FIG. 2 again, in step 205, the result output unit 115 may output the generated predicted recipe as a recipe that satisfies the target property and the constraint condition.

For example, the result output unit 115 may output the result recipe through an output unit (e.g., display) (not illustrated) of the device 100 or an output unit (e.g., display) of another device connected to the device 100, or may transmit the result recipe to another preset device.

At this time, the result output unit 115 may store the finally obtained plurality of predicted recipes in the storage unit 120 as recipes that satisfy the target property and the constraint condition.

In addition, the result output unit 115 may generate a report related to the generation of the predicted recipe, and output the generated result report.

To describe in more detail, the result output unit 115 may generate a result report including at least some information among various information such as the number of search areas derived during the operation process of the recipe prediction model 121, the number of grid points where feasible solutions exist, the number of grid points where feasible solutions do not exist, the number of the reduced plurality of search areas, the number of excluded search areas, the number of restored search areas, the maximum error, and the minimum error.

In addition, the result output unit 115 may predict the properties of the polymer composite material for each of the plurality of previously obtained predicted recipes, and calculate the matching rate or error rate (%) with the target property for at least one property item to be included in the result report.

In addition, the result output unit 115 may predict the properties of the polymer composite material for each of the plurality of previously obtained predicted recipes, and calculate an average matching rate or error rate (%) with the target property to be included in the result report.

In addition, the result output unit 115 may calculate an excess usage rate (e.g., an excess usage rate with respect to a minimum usage rate) for a specific polymer (such as virgin polymer or recycled polymer) based on a minimum usage rate (or a maximum usage rate) of a specific polymer (such as virgin polymer or recycled polymer) set in the constraint condition and a mixing ratio of the result recipe, to be included in the result report.

According to various embodiments, the result output unit 115 may include at least some chemical formulas and/or at least some chemical structures of the polymer composite material synthesized according to the predicted recipe in the result information. At this time, the result output unit 115 may display a part corresponding to the target property of the polymer composite material through highlighting or color change.

At this time, the result output unit 115 may predict the properties, at least some chemical formulas, and/or at least some chemical structures of the polymer composite material for the predicted recipe using the property prediction model 123.

The result processing unit 115 may store the generated result report and various information generated during the generation process of the result report in the storage unit 120.

The result output unit 115 may terminate the embodiment of FIG. 2 after the operation of step 205 is performed.

As described above, the method and the device for generating recipes of a polymer composite material have an effect of quickly deriving a recipe that matches the target property and the constraint condition by deriving a predicted recipe based on a Bayesian optimization algorithm applied in parallel to a plurality of targets (such as search areas).

According to various non-limiting embodiments or aspects, the method and the device for generating recipes of a polymer composite material have an effect of improving the recipe matching rate for the target property and the constraint condition by flexibly controlling the ranges of the search areas based on an error in the Bayesian optimization process.

According to various non-limiting embodiments or aspects, by determining search areas where an optimal solution exists using an objective function generated based on the result of applying a weight to each grid point of the plurality of search areas, the method and the device for generating recipes of a polymer composite material have an effect of reducing the situation where a recipe that does not match the actual target property is derived due to overfitting of the property prediction model pre-trained using artificial intelligence or due to prediction errors.

As described above, although the embodiments or aspects have been described with reference to the limited drawings, it will be apparent to those skilled in the art that various modifications and alternations may be applied thereto based on the various non-limiting embodiments or aspects.

For example, adequate effects or results may be achieved even if the foregoing processes and methods are carried out in different order than those described above, and/or the above-described elements, such as systems, structures, devices, or circuits, are combined or coupled in different forms and modes than those described above, or substituted or switched with other components or equivalents.

In some non-limiting embodiments or aspects, when describing with reference to the flowchart, it is described that a plurality of steps are configured and the steps are sequentially executed in a designated order, but it is not necessarily limited to the designated order.

In other words, executing by changing or deleting at least some of the steps described in the flowchart or adding at least one step is applicable in some non-limiting embodiments or aspects, and executing one or more steps in parallel may also be applicable in some non-limiting embodiments or aspects. That is, it is not limited to that the steps are necessarily operated in a time-series order, and should be comprised in various embodiments of the present disclosure.

Therefore, other implements, other embodiments or aspects, and equivalents to claims are within the scope of the present disclosure.

**Claims**

1.  A method for generating recipes of a polymer composite material, the method comprising:

    inputting a target property for at least two property items related to a target polymer composite material and a constraint condition for use of a material related to synthesis of the target polymer composite material;
    generating a predicted recipe for synthesis of the target polymer composite material using a recipe prediction model under the input target property and the constraint condition; and
    outputting the generated predicted recipe;
    wherein the predicted recipe comprises at least two materials and a mixing ratio for the at least two materials.

2.  The method according to claim 1, wherein the recipe prediction model is configured to generate the predicted recipe based on a property prediction model which is pre-trained using artificial intelligence and an optimization algorithm applied to the property prediction model, and

    the property prediction model is an artificial intelligence model trained to receive as an input, an input recipe comprising a plurality of materials comprising at least one polymer and a mixing ratio for the plurality of materials, and generate predicted properties of the polymer composite material according to an input of the input recipe, wherein in particular the artificial intelligence model is trained with a training data set including at least one training recipe for at least one training polymer composite material and training property information, said training recipe including information on materials including at least one polymer and mixing ratio of the materials satisfying training properties of each of the at least one training polymer composite material, said training properties including at least one of the following: melt index, tensile strength, tensile break, tearing strength, yield strength, flexural modulus, impact strength, elongation at break, heat distortion temperature, air permeability, and shrinkage.

3.  The method according to claim 2, wherein generating the predicted recipe comprises:

    generating a search space and a grid based on the search space for deriving candidate recipes based on the target property, the constraint condition, and the property prediction model;
    dividing the search space into a plurality of search areas based on materials having linearly approximable properties and the property prediction model;

reducing the plurality of search areas based on a preset weight; and

determining, as the predicted recipe, candidate recipes calculated by applying the optimization algorithm in parallel to each of the reduced plurality of search areas.

4. The method according to claim 3, wherein reducing the plurality of search areas comprises:

   determining search areas in which an optimal solution exists, using an objective function generated based on a result to which the preset weight is applied for a grid point of each of the plurality of search areas; and

   obtaining the reduced plurality of search areas configured with the search areas in which the optimal solution exists.

5. The method according to claim 4, wherein reducing the plurality of search areas comprises, changing at least some values of the constraint condition when a number of the search areas in which the optimal solution exists is less than a preset number.

6. The method according to anyone of claims 1-5, wherein the target property comprises at least one of a property value range for at least one property item or information on candidate materials.

7. The method according to anyone of claims 1-6, wherein the constraint condition comprises information on at least one of: at least one required material, a minimum usage ratio for each material, or a number of maximum usable materials.

8. The method according to anyone of claims 3-5, wherein determining, as the predicted recipe, candidate recipes further comprises:

   obtaining at least one updated objective function for deriving the candidate recipes during applying the optimization algorithm in parallel to each of the reduced plurality of search areas;

   calculating an error of the at least one updated objective function; and

   excluding, from the reduced plurality of search areas, a search area corresponding to an updated objective function having an error equal to or greater than a preset value.

9. A device for generating recipes of a polymer composite material, the device comprising:

   a property information processing unit (111) configured to input a target property for at least two property items related to a target polymer composite material and a constraint condition for use of a material related to synthesis of the target polymer composite material;

   a recipe prediction processing unit (113) configured to generate a predicted recipe for synthesis of the target polymer composite material using a recipe prediction model under the input target property and the constraint condition; and

   a result output unit (115) configured to output the generated predicted recipe;

   wherein the predicted recipe comprises at least two materials and a mixing ratio for the at least two materials.

10. The device according to claim 9, wherein the recipe prediction model is configured to generate the predicted recipe based on a property prediction model which is pre-trained using artificial intelligence and an optimization algorithm applied to the property prediction model, and

   the property prediction model is an artificial intelligence model trained to receive, as an input, an input recipe comprising a plurality of materials comprising at least one polymer and a mixing ratio for the plurality of materials, and to generate predicted properties of the polymer composite material according to the input of the input recipe.

11. The device according to claim 10, wherein the recipe prediction processing unit is configured to:

   generate a search space and a grid based on the search space for deriving candidate recipes based on the target property, the constraint condition, and the property prediction model;

   divide the search space into a plurality of search areas based on materials having linearly approximable properties and the property prediction model;

   reduce the plurality of search areas based on a preset weight; and

   determine, as the predicted recipe, candidate recipes calculated by applying the optimization algorithm in parallel to each of the reduced plurality of search areas.

**12.** The device according to claim 11, wherein the recipe prediction processing unit is configured to:

determine search areas in which an optimal solution exists, using an objective function generated based on a result to which the preset weight is applied for a grid point of each of the plurality of search areas; and obtain the reduced plurality of search areas configured with the search areas in which the optimal solution exists.

**13.** The device according to claim 12, wherein the recipe prediction processing unit is configured to change at least some values of the constraint condition when a number of the search areas in which the optimal solution exists is less than a preset number, while performing an operation of reducing the plurality of search areas.

**14.** The device according to anyone of claims 9-13, wherein the target property comprises at least one of a property value range for at least one property item or information on candidate materials.

**15.** The device according to anyone of claims 9-14, wherein the constraint condition comprises information on at least one of: at least one required material, a minimum usage ratio for each material, or a number of maximum usable materials.

[FIG. 1]

[FIG. 2]

[FIG. 3]

203

301

Generating A Search Space And An Initial Grid Based On The Search Space For Deriving Candidate Recipes Based On The Target Property, The Constraint Condition, And The Property Prediction Mode

303

Dividing The Search Space Into A Plurality Of Search Areas Based On Materials Having Linearly Approximable Properties And The Property Prediction Model

305

Reducing The Plurality Of Search Areas Based On A Preset Weighted Average

307

Determining The Resulting Recipes Calculated By Applying The Optimization Algorithm In Parallel To Each Of The Reduced Plurality Of Search Areas As The Predicted Recipe.

[FIG. 4]

305

401

Determining Search Areas In Which An Optimal Solution Exists, Using An Objective Function Generated Based On A Result To Which The Weighted Average Is Applied For A Grid Point Of Each Of The Plurality Of Search Areas

403

Obtaining The Reduced Plurality Of Search Areas Configured With The Search Areas In Which The Optimal Solution Exists

[FIG. 5]

**307**

**501**

Obtaining At Least One Updated Objective Function For Deriving
The Candidate Recipes During Applying The Optimization Algorithm
In Parallel To Each Of The Reduced Plurality Of Search Areas

**503**

Calculating An Error Of The Updated Objective Function

**505**

Exclude, From The Reduced Plurality Of Search Areas, A Search
Area Corresponding To An Updated Objective Function Having
An Error Equal To Or Greater Than A Preset Second Error

[FIG. 6]

Grid Point Where A Feasible Solution Exists
Grid Point Where No Feasible Solution Exists
Searched Area
Optimal Solution Found For Each Search Area

[FIG. 7]

Europäisches
Patentamt

European
Patent Office

Office européen
des brevets

# EUROPEAN SEARCH REPORT

**Application Number**

EP 25 19 2687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/185609 A1 (SUMITOMO CHEMICAL CO [JP]) 12 September 2024 (2024-09-12) | 1,2,6,7, 9,10,14, 15 | INV. G16C60/00 |
| A | * the whole document * | 3-5,8, 11-13 | ADD. G16C20/10 G16C20/30 G16C20/70 |
| X | WO 2023/088604 A1 (DSM IP ASSETS BV [NL]) 25 May 2023 (2023-05-25) | 1,9 | |
| A | * claims 1,8 * * figures 1,2,3A * * page 12 - page 13 * | 2-8, 10-15 | |
| A | JP 2024 070636 A (SUMITOMO CHEMICAL CO) 23 May 2024 (2024-05-23) * the whole document * | 1-15 | |
| A | US 11 837 333 B1 (SHUANG BO [US] ET AL) 5 December 2023 (2023-12-05) * the whole document * | 1-15 | |

**TECHNICAL FIELDS SEARCHED (IPC)**

G16C

The present search report has been drawn up for all claims

| Place of search | Date of completion of the search | Examiner |
|---|---|---|
| The Hague | 14 January 2026 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 2687

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2026

| Patent document cited in search report | | Publication date | Patent family member(s) | | Publication date |
|---|---|---|---|---|---|
| WO 2024185609 | A1 | 12-09-2024 | EP | 4679433 A1 | 14-01-2026 |
| | | | JP | 7352758 B1 | 28-09-2023 |
| | | | JP | 7382533 B1 | 16-11-2023 |
| | | | JP | 7426531 B1 | 01-02-2024 |
| | | | JP | 2024127096 A | 20-09-2024 |
| | | | JP | 2024127708 A | 20-09-2024 |
| | | | JP | 2024127709 A | 20-09-2024 |
| | | | WO | 2024185609 A1 | 12-09-2024 |
| WO 2023088604 | A1 | 25-05-2023 | CN | 118235133 A | 21-06-2024 |
| | | | EP | 4433937 A1 | 25-09-2024 |
| | | | JP | 2024546017 A | 17-12-2024 |
| | | | KR | 20240095465 A | 25-06-2024 |
| | | | US | 2025013920 A1 | 09-01-2025 |
| | | | WO | 2023088604 A1 | 25-05-2023 |
| JP 2024070636 | A | 23-05-2024 | JP | 7407893 B1 | 04-01-2024 |
| | | | JP | 2024070636 A | 23-05-2024 |
| US 11837333 | B1 | 05-12-2023 | AU | 2023409200 A1 | 03-07-2025 |
| | | | CA | 3276527 A1 | 27-06-2024 |
| | | | CN | 120322827 A | 15-07-2025 |
| | | | EP | 4612695 A1 | 10-09-2025 |
| | | | IL | 320885 A | 01-07-2025 |
| | | | US | 11837333 B1 | 05-12-2023 |
| | | | WO | 2024137021 A1 | 27-06-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240129283